# EUROPÄISCHE PATENTSCHRIFT

(11) **EP 1 773 486 B1**
(45) Veröffentlichungstag und Bekanntmachung des Hinweises auf die Patenterteilung: **08.09.2010**
(21) Anmeldenummer: 05761020.6
(22) Anmeldetag: 05.07.2005
(51) Int. Cl.: B01J 23/30

(54) **WOLFRAMAT ENTHALTENDE KATALYSATOREN ZUR SYNTHESE VON ALKYLMERCAPTAN UND VERFAHREN ZU IHRER HERSTELLUNG**
CATALYSTS CONTAINING TUNGSTATE FOR THE SYNTHESIS OF ALKYLMERCAPTANE AND METHOD FOR THE PRODUCTION THEREOF
CATALYSEURS RENFERMANT UN WOLFRAMATE POUR SYNTHETISER DES ALKYLMERCAPTANS, ET PROCEDE DE PRODUCTION DE CES CATALYSEURS

(30) Priorität: 04.08.2004 DE 102004037739
(43) Veröffentlichungstag der Anmeldung: 18.04.2007
(73) Patentinhaber: Evonik Degussa GmbH, 45128 Essen (DE)
(72) Erfinder: REDLINGSHÖFER, Hubert, 91481 Münchsteinach (DE); WECKBECKER, Christoph, 63584 Grundau-Lieblos (DE); HUTHMACHER, Klaus, 63571 Geinhausen (DE)
(86) Internationale Anmeldenummer: PCT/EP2005/007211
(87) Internationale Veröffentlichungsnummer: WO 2006/015668

(56) Entgegenhaltungen:
- EP-A- 0 832 687
- EP-A- 0 832 878
- WO-A-2005/021491
- MASHKINA A V ET AL: "ACTIVITY OF TUNGSTATE CATALYSTS IN THE SYNTHESIS OF METHYL - MERCAPTANE FROM METHANOL AND HYDROGEN SULFIDE" REACTION KINETICS AND CATALYSIS LETTERS, ELSEVIER, AMSTERDAM, NL, Bd. 36, Nr. 1, 1988, Seiten 159-164, XP002063476 ISSN: 0133-1736 in der Anmeldung erwähnt

## Beschreibung

Die vorliegende Erfindung betrifft einen Kaliwolframat enthaltenden Katalysator zur Synthese von Alkylmercaptanen aus Alkanolen und Schwefelwasserstoff.

Insbesondere Methylmercaptan ist ein industriell wichtiges Zwischenprodukt zum Beispiel für die Synthese von Methionin sowie für die Synthese von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drücken zwischen 1 und 25 bar.

Das Reaktionsgemisch enthält neben dem gebildeten Methylmercaptan die nicht umgesetzten Ausgangsstoffe und Nebenprodukte, wie zum Beispiel Dimethylsulfid und Dimethylether sowie die im Sinne der Reaktion inerten Gase, wie zum Beispiel Methan, Kohlenmonoxid, Wasserstoff und Stickstoff. Aus diesem Reaktionsgemisch wird das gebildete Methylmercaptan abgetrennt.

Für die Wirtschaftlichkeit des Verfahrens wird eine möglichst hohe Selektivität bei der katalytischen Reaktion von Methanol und Schwefelwasserstoff zu Methylmercaptan gefordert, um den Aufwand bei der Abtrennung des gebildeten Methylmercaptans vom Reaktionsgemisch so gering wie möglich zu halten. Hier stellt insbesondere der Energieaufwand zur Kühlung des Reaktionsgasgemisches zur Kondensation des Methylmercaptans einen großen Kostenfaktor dar.

Zur Erhöhung von Aktivität und Selektivität wird Aluminiumoxid als Träger üblicherweise mit Kaliumwolframat oder Cäsiumwolframat versetzt. Dabei wird das Wolframat gewöhnlich in Mengen bis 25 Gew.-%, bezogen auf das Gesamtgewicht des Katalysators, eingesetzt. Eine Verbesserung von Aktivität und Selektivität erhält man auch durch Erhöhung des Molverhältnisses von Schwefelwasserstoff zu Methanol. Üblicherweise werden Molverhältnisse zwischen 1 und 10 angewendet.

Ein hohes Molverhältnis bedeutet allerdings auch einen hohen Überschuss des Schwefelwasserstoffes im Reaktionsgemisch und somit die Notwendigkeit, große Gasmengen im Kreis zu führen. Zur Verminderung des hierfür benötigten Energieaufwandes sollte daher das Verhältnis von Schwefelwasserstoff zu Methanol nur wenig von 1 abweichen.

Die US-PS 2,820,062 betrifft ein Verfahren zur Herstellung von organischen Thiolen, bei welchem ein Katalysator aus aktivem Aluminiumoxid verwendet wird, der mit Kaliumwolframat in einer Menge von 1,5 bis 15 Gew.-%, bezogen auf das Gewicht des Katalysators, versetzt ist. Mit diesem Katalysator werden gute Aktivitäten und Selektivitäten bei Reaktionstemperaturen von 400°C und Molverhältnissen von 2 erzielt. Diese US-Patentschrift nennt verschiedene Möglichkeiten zur Einbringung des Kaliumwolframats in das Aluminiumoxid. So sollen Imprägnierverfahren, Copräzipitationen und reine Mischungen anwendbar sein. Der eigentlichen Herstellung des Katalysators wird wenig Bedeutung für die Wirtschaftlichkeit des Syntheseverfahrens von Methylmercaptan eingeräumt.

In der EP 0 832 687 B1 werden die Vorteile der Verwendung von Cäsiumwolframat (Cs₂WO₄) anstelle von Kaliumwolframat (K₂WO₄) als Promotor beschrieben. So kann durch Einsatz von Cäsiumwolframat eine gesteigerte Aktivität bei gleichzeitig guter Selektivität erreicht werden.

Durch Erhöhung der Cäsiumwolframat-Konzentration auf bis zu 40 Gew.-% kann die Selektivität zu Methylmercaptan auf bis zu 92 % gesteigert werden, ohne dass die Aktivität unverhältnismäßig verschlechtert wird.

Nach allgemeiner Ansicht erzielt man die beste Selektivität mit Katalysatoren, bei denen das Alkali/Wolframverhältnis gleich 2:1 ist (A.V. Mashkina et al., React. Kinet. Catal. Lett., Vol. 36, No. 1, 159-164 (1988).

Die EP 0 832 878 A2 offenbart in einer bevorzugten Ausführungsform einen Kaliumwolframat enthaltenden Katalysator, in dem sich das Kalium/Wolframverhältnis auf 2:1 beläuft.

Die am 10.03.2005 veröffentlichte WO 2005/021491 mit der Priorität vom 23.08.2003 betrifft einen oxidischen, Cäsium und Wolfram enthaltenden Katalysator zur Synthese von Alkylmercaptanen aus Alkanolen und Schwefelwasserstoff, sowie ein Verfahren zur Herstellung dieses Katalysators, wobei das molare Verhältnis Cäsium zu Wolfram < 2:1 ist.

Aufgabe der vorliegenden Erfindung ist es, einen Katalysator zur Verfindung zu stellen, welcher sich bei niedrigen Molverhältnissen von Schwefelwasserstoff zu Methanol durch verbesserte Aktivität und Selektivität gegenüber den bekannten Katalysatoren auszeichnet und somit zu einer besseren Wirtschaftlichkeit des Verfahrens führt.

Diese Aufgabe wird durch die Bereitstellung eines Katalysators gelöst, enthaltend eine katalytisch aktives Alkaliwolframat, welches gebundene Alkalimetalle Kalium oder Kalium und Cäsium und Wolfram mit einem molaren Verhältnis von Alkalimetallen zu Wolfram von 1,9:1 bis 1:1, besonders 1,6:1 bis 1:1, enthält.

Die oxidische Zusammensetzung lässt sich mit der Formel AₓWO_{y} beschreiben, in der A für Alkalimetall steht und x:< 2 bis 0,9 und y:3,4 bis < 4 bedeuten.

Der Katalysator enthält das Wolframat in einer Menge von 8 bis 45 Gew.-%, insbesondere 20 bis 36 Gew.-%, bevorzugt > 25 bis 36 Gew.-%. Im Falle eines Schalenkatalysators beziehen sich diese Anteile auf die Zusammensetzung der Schale.

Die oxidischen Verbindungen aus den genannten Alkalimetall(en) und Wolfram könnendirekt auf einen Trägerkörper imprägniert werden (Trägerkatatlysator).

Bei der Herstellung von Katalysatoren in Form von Extrudaten oder Preßlingen wird der pulverförmige Träger mit der oxidischen Zusammensetzung imprägniert oder vermischt und die erhaltene Zwischenstufe anschließend verformt (Vollkatalysator). Wird ein Schalenkatalysator hergestellt, so wird der pulverförmigen Träger mit der katalytisch wirksamen Zusammensetzung imprägniert und die entstehende Mischung dann auf einen bevorzugt inerten Trägerkern in Form einer Schale aufgebracht wird.

Das Alkali/W-Verhältnis beläuft sich auf 1, 9:1 bis 1:1. Damit enthalten die erfindungsgemäßen Katalysatoren für die Umsetzung von Alkanolen mit Schwefelwasserstoff zu Alkylmercaptanen einen im Vergleich zum mit Cäsiumwolframat (Cs₂WO₄) oder Kaliumwolframat (K₂WO₄) imprägnierten Katalysator gemäss Stand der Technik überstöchiometrischen Anteil an Wolfram.

Es zeigt sich, dass dieser höhere Anteil im Wolframat auf dem bevorzugt eingesetzten Aluminiumoxid im Vergleich zu dem im Stand der Technik ausschließlich verwendeten stöchiometrischen Alkaliwolframat dem Katalysator eine verbesserte Aktivität bei gleichzeitig verbesserter Selektivität verleiht. Während die Erhöhung der Konzentration von Cäsiumwolframat (Cs₂WO₄) auf dem Katalysator lediglich eine Steigerung der Selektivität bei gleichzeitig geringerer Aktivität bewirkt, zeigt sich bei der Erhöhung des Wolframgehalts im Verhältnis zum Alkaligehalt in unerwarteter Weise eine weitere Steigerung der Selektivität bei gleichzeitig gesteigerter Aktivität. Erfindungsgemäß kann bei sehr hohen Beladungen mit dem Promotor eine ausgezeichnete Selektivität erreicht werden, ohne dass die Aktivität des Katalysators, wie aus dem Stand der Technik bekannt, abnimmt. Weiterhin wurde gefunden, dass über das Alkali-Wolfram-Verhältnis und über die Wahl der Alkalimetalle die Aktivität und Selektivität des Katalysators gezielt eingestellt werden kann. Bei der Verwendung von Mischungen von Alkalimetallen können zudem die vergleichsweise teureren Metalle wie Cäsium zumindest teilweise durch kostengünstigere wie Kalium ersetzt werden, ohne dass die Aktivität oder Selektivität des Katalysators beeinträchtigt wird.

Der Katalysator wird in Form eines Trägerkatalysators, bei dem die Oberfläche mit der katalytisch wirksamen Substanz imprägniert wird, oder eines Schalenkatalysators, bei dem ein bevorzugt inerter Kern mit einem Gemisch aus katalytisch aktiver Substanz und Trägermaterial umgeben ist, eingesetzt. Weiterhin können Extrudate oder Preßlinge, bei denen die katalytisch aktive Substanz mit dem pulverförmigen Trägermaterial vor der Verformung vermischt bzw. diese mit ihr imprägniert wird, verwendet werden. Als Trägermaterialien werden die bekannten oxidischen anorganischen Verbindungen wie zum Beispiel SiO₂, TiO₂, ZrO₂ und bevorzugt sogenanntes aktives Aluminiumoxid eingesetzt. Dieses Material weist hohe spezifische Oberflächen zwischen etwa 10 und 400 m²/g auf und besteht hauptsächlich aus Oxiden der Übergangsreihe der kristallographischen Phasen des Aluminiumoxids (siehe zum Beispiel Ullmann's Enzyclopedia of Industrial Chemistry von 1985, Vol. Al, Seiten 561-562). Zu diesen Übergangsoxiden gehören γ-, δ-, η-, κ-, χ- und θ-Aluminiumoxid. Alle diese kristallographischen Phasen gehen bei Erhitzung des Aluminiumoxids auf Temperaturen über 1100°C in das thermisch stabile α-Aluminiumoxid über. Aktives Aluminiumoxid wird kommerziell für katalytische Anwendungen in verschiedenen Qualitäten und Lieferformen angeboten. Besonders geeignet für die Herstellung von Trägerkatalysatoren sind Formkörper aus granuliertem oder stranggepresstem Aluminiumoxid mit Korndurchmessern von 1 bis 5 mm, einer spezifischen Oberfläche von 180 bis 400 m²/g, einem Gesamtporenvolumen zwischen 0,3 und 1,2 ml/g sowie einer Schüttdichte von 300 bis 900 g/l. Für die Zwecke der Erfindung wird bevorzugt Aluminiumoxid mit mehr als 200 m²/g spezifischer Oberfläche verwendet, da die katalytische Aktivität des fertigen Katalysators mit steigender Oberfläche des Aluminiumoxids leicht ansteigt. Dieses Material wird in Pulverform bevorzugt für die Herstellung der Schalenkatalysatoren, Extrudate oder Preßlinge eingesetzt.

Die wässrige Imprägnierlösung zur Aufbringung des Promotors kann in einfacher Weise aus in Wasser löslichen Alkali- und Wolframverbindungen, insbesondere Wolframsäure (H₂WO₄) und Alkalihydroxiden hergestellt werden. Hierzu wird z. B. Wolframsäure in Wasser suspendiert und unter Zugabe einer Base und Erwärmen aufgelöst. Alkalihydroxid oder ein anderes Alkalisalz wird ebenfalls in Wasser aufgelöst und mit der Lösung der Wolframsäure vereinigt (Promotorlösung). Vorteilhaft einsetzbar sind aber auch Alkalialze, deren Anionen sich durch Wärmebehandlung rückstandslos austreiben lassen wie z. B Nitrate, Formiate, Oxalate, Acetate oder Carbonate. Zur Stabilisierung dieser Lösung mit einem pH von 8 bis 14 eignen sich anorganische und auch organische Basen. Bevorzugt werden solche Basen eingesetzt, die sich durch eine abschließende Wärmebehandlung des nach der Imprägnierung erhaltenen Katalysators rückstandslos austreiben lassen. Zu diesen Basen gehören bevorzugt Ammoniumhydroxid und organische Basen, insbesondere Amine. Gegenüber dem Stand der Technik wird das molare Verhältnis von Alkalimetallen und W beim Ansetzen der wässrigen Imprägnierlösung derart gewählt, dass im Unterschied zu Cäsiumwolframat (Cs₂WO₄) oder Kaliumwolframat (K₂WO₄) mit einem Alkali/W-Verhältnis von 2 zu 1 ein höherer Anteil an Wolfram, d.h. ein Alkali zu W-Verhältnis 1,9:1 bis 1:1 vorliegt. Dies führt im Vergleich zu den bekannten Katalysatoren zu einer deutlich erhöhten Aktivität und Selektivität der erfindungsgemäßen Katalysatoren, insbesondere bei niedrigen Verhältnissen von Schwefelwasserstoff und Methanol im Reaktionsgas.

Bei der Verwendung von Mischungen von Wolframaten mit gemischten Alkalianteilen handelt es sich um die Alkalimetalle Kalium und Cäsium in einem Verhältnis zwischen 0,01:1,0 und 1,0:1,0. Dabei wird der Anteil des kostengünstigeren Alkalimetalls bevorzugt so weit gesteigert und gleichzeitig derjenige des vergleichsweise teureren Alkalimetalls im Gegenzug verringert, dass keine Verschlechterung der Aktivität oder Selektivität des Katalysators eintritt.

Für das Aufbringen der Promotorlösung können verschiedene Imprägniertechniken wie das Tauchimprägnieren, Sprühimprägnieren, Vakuumimprägnieren und die Porenvolumenimprägnierung eingesetzt werden, wobei die Imprägnierung auch mehrfach erfolgen kann. Bei Formkörpern muss das gewählte Imprägnierverfahren es ermöglichen, die gewünschte Beladungsmenge des Promotors mit guter Gleichmäßigkeit über den gesamten Querschnitt aufzubringen.

Bevorzugt wird die Promotorlösung durch Sprüh- oder Vakuumimprägnierung in einem oder in zwei Schritten auf die Formkörper aufgebracht. Bei der Sprühimprägnierung wird die wässrige Imprägnierlösung auf die Trägerkörper gesprüht. Bei der Vakuumimprägnierung wird in einem mit den Formkörpern gefüllten Behälter mittels einer Vakuumpumpe ein Unterdruck erzeugt. Durch Öffnen einer Schlauchverbindung zur wässrigen Imprägnierlösung wird die Lösung in den Behälter gesaugt, bis die gesamte Schüttung der Formkörper mit der Lösung bedeckt ist. Nach einer Imprägnierzeit von 0,2 bis 2 Stunden wird die nicht durch das Material aufgenommene Lösung abgelassen oder abgegossen.

Durch Vortrocknung für die Dauer von 1 bis 10 Stunden bei Raumtemperatur kann sich das anfängliche Konzentrationsgefälle über den Querschnitt der Formkörper weitgehend ausgleichen. Somit wird die Gleichmäßigkeit der Imprägnierung über den Querschnitt der Katalysatorpartikel verbessert. Bevorzugt werden die so erhaltenen Katalysatorvorstufen für die Dauer von 1 bis 10 Stunden bei 100 bis 200, bevorzugt 100 bis 140°C zur Entfernung der Restfeuchte getrocknet. Dann erfolgt für die Dauer von 1 bis 20, bevorzugt 1 bis 5 Stunden eine Kalzinierung bei 300 bis 600, bevorzugt 420 bis 480°C. Dadurch wird der Promotor auf dem Aluminiumoxid fixiert und die Base der Imprägnierlösung zersetzt und ausgetrieben. Optional kann die Schüttung der Trägerkörper der Katalysatorvorstufen bei der Vortrocknung, Trocknung und Kalzinierung von einem Gasstrom durchströmt werden, was den Abtransport der Restfeuchte und der Zersetzungsgase verbessert.

Die Imprägnierung der Formkörper kann auch mehrstufig, insbesondere zweistufig erfolgen.

In einer weiteren Ausführungsform enthält dann die in der ersten Stufe eingesetzte Lösung ein bis zu zwei Drittel der vorgesehenen Gesamtmenge an Alkali- und Wolframverbindungen.

Geht man mehrstufig, mindestens aber zweistufig vor, kalziniert man die im ersten Schritt erhaltene Vorstufe gegebenenfalls nicht.

Ansonsten läuft in der zweiten Stufe dasselbe Imprägnier-, Trocknungs- und Kalzinierungsprogramm wie für das einstufige Verfahren beschrieben ab.

Diese mehrstufige Imprägnierung ist vor allem dann sinnvoll, wenn hohe Beladungen gewünscht werden und/oder die begrenzte Löslichkeit der Promotormischung die Beladung in einem Schritt nicht ermöglichen.

Es besteht auch die Möglichkeit, die Trägerkörper während des Imprägniervorgangs (Schritt a aus Anspruch 11) mehrfach mit der Imprägnierlösung zu besprühen und zwischen diesen Behandlungsschritten jeweils Teile der Restfeuchte bei einer Temperatur von bis zu 120°C zu entfernen, bevor man zu Schritt b übergeht.

Bei der Herstellung des Schalenkatalysators kann das als Schale aufzubringende Pulver vor oder nach der Beschichtung kalziniert werden. Beispielsweise kann dieser Katalysatortyp gemäß EP-B-0 068 193 hergestellt werden. Auch bei der Herstellung der Extrudate oder der Preßlinge kann die Kalzinierung vor und/oder nach der Verformung erfolgen.

### Beispiele

### Beispiel 1 (Vergleichsbeispiel)

150 g Aluminiumoxid I wurde mit 21,0 Gew.-% Cäsiumwolframat (Cs_{2,0}WO₄) mit Hilfe der Vakuumimprägnierung imprägniert. Hierzu wurde im einzelnen wie folgt vorgegangen:

Zur Herstellung der Imprägnierlösung wurden 55,7 g Wolframsäure in 44,5 g Wasser suspendiert und durch Zugabe von 111,4 g 25 %-iger Ammoniaklösung und Erwärmen auf 50°C gelöst. 74,6 g Cs(OH)·H₂O wurden in 37,3 g Wasser gelöst und mit der ersten Lösung vermischt. Die Lösung wurde anschließend im abgedeckten Becherglas 48 Stunden gerührt. Daraufhin wurde die Lösung mit 25 g Wasser auf ein Volumen von 234 ml aufgefüllt.

Das Aluminiumoxid wurde in einem Glasgefäß, das auf 150 mbar evakuiert wurde, vorgelegt. Durch das Öffnen eines Hahns wurde die Imprägnierlösung so lange in das evakuierte Glasgefäß gesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 79 ml Imprägnierlösung aufgenommen.

Das Granulat wurde für die Dauer von 1 Stunde bei Raumtemperatur im Luftstrom und anschließend bei 120°C für 3 Stunden zur Entfernung der Restfeuchte getrocknet. Danach wurde das Granulat 3 Stunden lang bei 455°C kalziniert.

### Beispiel 2 (Vergleichsbeispiel)

Vergleichsbeispiel 1 wurde mit 26,3 %-iger Beladung des Aluminiumoxids mit Cäsiumwolframat (cs_{2,0}WO₄) wiederholt.

### Beispiel 3 (Vergleichsbeispiel)

Vergleichsbeispiel 1 wurde mit 19,6 %-iger Beladung des Aluminiumoxids mit Kaliumwolframat (K_{2,0}WO₄) unter Verwendung von KOH statt Cs(OH)·H₂O wiederholt.

### Beispiel 4

150 g Aluminiumoxid (Spheralite 501A) wurden in einer zweistufigen Imprägnierung mit insgesamt 26,7 Gew.-% Promotor (K_{1,6}WO_{y}) mit Hilfe der Vakuumimprägnierung imprägniert. Im einzelnen wurde wie folgt vorgegangen:

64,5 g Wolframsäure wurden in 50,7 g Wasser suspendiert und durch Zugabe von 126,9 g 25 %-iger Ammoniaklösung und Erwärmen auf 50°C gelöst. 22,8 g KOH wurden in 11,5 g Wasser gelöst und mit der ersten Lösung vermischt. Die Lösung wurde anschließend im abgedeckten Becherglas 48 Stunden gerührt. Daraufhin wurde die Lösung mit 39 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 76 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

Zur Durchführung der zweiten Imprägnierung wurde eine gleiche Imprägnierlösung wie beim ersten Schritt angesetzt und in gleicher Weise durch Vakuumimprägnierung auf den bereits beladenen Katalysator aus dem ersten Schritt aufgebracht. Dann schloss sich wieder eine 1-stündige Trocknung bei Raumtemperatur an, gefolgt von einer 3-stündigen Trocknung bei 120°C. Abschließend wurden die Katalysatorpartikel 4 Stunden bei 455°C an Luft kalziniert.

### Beispiel 5 (Kein Teil der Erfindung)

150 g Aluminiumoxid (Spheralite 501A) wurden in einer zweistufigen Imprägnierung mit insgesamt 30,1 Gew.-% Promotor (Rb_{0,9}WO_{y}) mit Hilfe der Vakuumimprägnierung imprägniert. Im einzelnen wurde wie folgt vorgegangen:

59,0 g Wolframsäure wurden in 48,3 g Wasser suspendiert und durch Zugabe von 110,7 g 25 %-iger Ammoniaklösung und Erwärmen auf 50°C gelöst. 41,5 g RbOH wurden in 17,5 g Wasser gelöst und mit der ersten Lösung vermischt. Die Lösung wurde anschließend im abgedeckten Becherglas 48 Stunden gerührt. Daraufhin wurde die Lösung mit 25 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 75 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

Zur Durchführung der zweiten Imprägnierung wurde eine gleiche Imprägnierlösung wie beim ersten Schritt angesetzt und in gleicher Weise durch Vakuumimprägnierung auf den bereits beladenen Katalysator aus dem ersten Schritt aufgebracht. Dann schloss sich wieder eine 1-stündige Trocknung bei Raumtemperatur an, gefolgt von einer 3-stündigen Trocknung bei 120°C. Abschließend wurden die Katalysatorpartikel 4 Stunden bei 455°C an Luft kalziniert.

### Beispiel 6

150 g Aluminiumoxid (Spheralite 501A) wurden in einer zweistufigen Imprägnierung mit insgesamt 29,4 Gew.-% Promotor (K_{0,7}Cs_{0,7}WO_{y}) mit Hilfe der Vakuumimprägnierung imprägniert. Im einzelnen wurde wie folgt vorgegangen:

61,3 g Wolframsäure wurden in 49,1 g Wasser suspendiert und durch Zugabe von 122,7 g 25 %-iger Ammoniaklösung und Erwärmen auf 50°C gelöst. 9,8 g KOH und 29,0 g Cs(OH)·H₂O wurden in 14,5 g Wasser gelöst und mit der ersten Lösung vermischt. Die Lösung wurde anschließend im abgedeckten Becherglas 48 Stunden gerührt. Daraufhin wurde die Lösung mit 47 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 75 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

Zur Durchführung der zweiten Imprägnierung wurde eine gleiche Imprägnierlösung wie beim ersten Schritt angesetzt und in gleicher Weise durch Vakuumimprägnierung auf den bereits beladenen Katalysator aus dem ersten Schritt aufgebracht. Dann schloss sich wieder eine 1-stündige Trocknung bei Raumtemperatur an, gefolgt von einer 3-stündigen Trocknung bei 120°C. Abschließend wurden die Katalysatorpartikel 4 Stunden bei 455°C an Luft kalziniert.

### Beispiel 7 (Kein Teil der Erfindung)

150 g Aluminiumoxid (Spheralite 501A) wurden in einer zweistufigen Imprägnierung mit insgesamt 31,0 Gew.-% Promotor (Na_{0,3}Cs_{1,1}WO_{y}) mit Hilfe der Vakuumimprägnierung imprägniert. Im einzelnen wurde wie folgt vorgegangen:

61,1 g Wolframsäure wurden in 48,9 g Wasser suspendiert und durch Zugabe von 122,1 g 25 %-iger Ammoniaklösung und Erwärmen auf 50°C gelöst. 3,2 g NaOH und 44,6 g Cs(OH)·H₂O wurden in 22,3 g Wasser gelöst und mit der ersten Lösung vermischt. Die Lösung wurde anschließend im abgedeckten Becherglas 48 Stunden gerührt. Daraufhin wurde die Lösung mit 40 g Wasser auf ein Volumen von 234 ml aufgefüllt. Das Aluminiumoxid wurde in einem Glasgefäß, das auf 150 mbar evakuiert wurde, vorgelegt. Durch das Öffnen eines Hahns wurde die Imprägnierlösung so lange angesaugt bis die gesamte Schüttung der Formkörper mit der Lösung bedeckt war. Nach einer Wartezeit von 15 Minuten und Belüftung des Glasgefäßes floss die nicht durch das Aluminiumoxid aufgenommene Lösung zurück in das Becherglas. Vom Aluminiumoxid wurden dabei 74 ml Imprägnierlösung aufgenommen. Anschließend wurde das Granulat 1 Stunde bei Raumtemperatur und 3 Stunden bei 120°C getrocknet sowie 3 Stunden bei 455°C kalziniert.

Zur Durchführung der zweiten Imprägnierung wurde eine gleiche Imprägnierlösung wie beim ersten Schritt angesetzt und in gleicher Weise durch Vakuumimprägnierung auf den bereits beladenen Katalysator aus dem ersten Schritt aufgebracht. Dann schloss sich wieder eine 1-stündige Trocknung bei Raumtemperatur an, gefolgt von einer 3-stündigen Trocknung bei 120°C. Abschließend wurden die Katalysatorpartikel 4 Stunden bei 455°C an Luft kalziniert.

### Beispiel 8 (Anwendungsbeispiel)

Die Katalysatoren wurden bezüglich ihrer Leistungsdaten bei der Synthese von Methylmercaptan aus Schwefelwasserstoff und Methanol getestet.

Die Synthese wurde in einem Edelstahlrohr von 18 mm Innendurchmesser und einer Länge von 500 mm durchgeführt. Die Katalysatorschüttung von jeweils 76 ml wurde beiderseits durch Inertschüttungen aus Glaskugeln im Reaktionsrohr fixiert. Das Reaktionsrohr wurde über einen Doppelmantel mit einem Thermoöl auf die Reaktionstemperatur von etwa 320°C aufgeheizt.

Die Versuchsbedingungen sind der folgenden Aufstellung zu entnehmen:

| | |
|---|---|
| GHSV: | 1300 h⁻¹ (bezogen auf Normbedingungen) |
| LHSV: | 0,84 h⁻¹ (bezogen auf flüssiges MeOH) |
| Reaktionstemperatur: | 320°C |
| Massenverhältnis | |
| H₂S/MeOH: | 1,9 |
| Druck: | 9 bar |

Das Reaktionsgemisch mit den Produkten Methylmercaptan, Dimethylsulfid und Dimethylether und mit den nicht umgesetzten Eingangsstoffen Methanol und Schwefelwasserstoff wird durch online Gaschromatographie analysiert.

Wird der Wolframanteil im Verhältnis zum Alkalianteil im Katalysator erhöht, so ist eine deutliche Steigerung der Aktivität bei gleichzeitig verbesserter Selektivität zu erkennen. Dies führt im Vergleich zum Stand der Technik zu einer Ausbeutesteigerung von bis zu 10 %. Die Selektivität kann durch Einstellung des Alkali-Wolframat-Verhältnisses auf bis ∼ 96,5 % gesteigert werden, wobei der Methanolumsatz ansteigt. Dies führt bei der großtechnischen Synthese von Methylmercaptan auch zu erheblichen Kosteneinsparungen bei der Abtrennung des Reaktionsproduktes von nicht umgesetztem Methanol und Nebenprodukten.

Weiterhin zeigen die Ergebnisse der Beispiele 4 bis 7, dass zumindest ein Teil der Alkalimetalle gegeneinander ausgetauscht werden kann, um die Aktivität und Selektivität des Katalysators gezielt einzustellen oder um Rohstoffkosten bei der Katalysatorfertigung einzusparen.

**Tabelle 1: Versuchsergebnisse**

| Katalysator Beispiel | Alkali-metall | mol-Verhält. Alkali:W | Beladung [Gew.-%] | Methanol-umsatz [%] | Selektivität [%] | Ausbeute [%] |
|---|---|---|---|---|---|---|
| VB1 | Cs | 2:1 | 21,0 | 82,4 | 93,3 | 76,9 |
| VB2 | Cs | 2:1 | 26,3 | 79,5 | 94,7 | 75,2 |
| VB3 | K | 2:1 | 19,6 | 76,0 | 95,2 | 72,4 |
| B4^{*)} | K | 1,6:1 | 26,7 | 85,6 | 95,1 | 81,4 |
| B5^{*)} | Rb | 0,9:1 | 30,1 | 73,2 | 96,6 | 70,7 |
| B6^{*)} | K, Cs | 1,4:1 | 29,4 | 88,5 | 95,4 | 84,4 |
| B7^{*)} | Na, Cs | 1,4:1 | 31,0 | 88,4 | 95,8 | 84,7 |

| | | | | | | |
|---|---|---|---|---|---|---|
| VB1: Katalysator nach Vergleichsbeispiel 1 ^{*)} mehrstufige Imprägnierung B5, B7 sind kein Teil der Erfindung | | | | | | |

## Patentansprüche

1. Katalysator, enthaltend ein katalytisch aktives Wolframat, welches als chemisch gebundenes Alkalimetall Kalium oder Kalium und Cäsium und Wolfram mit einem molaren Verhältnis von Alkalimetall zu Wolfram von 1,9:1 bis 1:1 enthält.

2. Katalysator gemäss den Anspruch 1, bestehend aus einem Schalenkatalysator, bei dem ein Trägerkern mit dem katalytischen aktiven Wolframat oder mit einem mit diesem Wolframat imprägnierten Trägermaterial umhüllt ist.

3. Katalysator gemäss Anspruch 1, bei dem das mit dem katalytisch aktiven Wolframat imprägnierte Trägermaterial zu einem Vollkatalysator verarbeitet worden ist.

4. Katalysator gemäss Anspruch 1, bei dem die Oberfläche eines Tägerkörpers mit dem genannten katalytisch aktiven Alkaliwolframat imprägniert ist.

5. Katalysator gemäß Anspruch 1, **dadurch gekennzeichnet, dass** sich das Verhältnis auf 1,6:1 bis 1:1 beläuft.

6. Katalysator gemäss einem oder mehreren der Ansprüche 1 bis 5, **dadurch** gekennziechnet, dass er das Wolframat in einer Menge von 8 bis 45 Gew.-%, bevorzugt 20 bis 36 Gew.-%, enthält.

7. Katalysator gemäss einem oder mehreren der Ansprüche 1 bis 6, **dadurch gekennzeichnet, dass** der Trägerkörper oder das Trägermaterial aus einer oxidischen anorganischen Verbindung besteht.

8. Katalysator gemäss Anspruch 7, **dadurch gekennzeichnet, dass** der Trägerkörper oder das Trägermaterial aus Aluminiumoxid (Al₂O₃) besteht.

9. Katalysator gemäss Anspruch 7, **dadurch gekennzeichnet, dass** das Trägermaterial eine spezifische Oberfläche von 180 bis 400 m²/g (BET) und ein Gesamtporenvolumen von 0,3 bis 1,2 ml/g.

10. Verfahren zur Herstellung von Alkylmercaptanen durch Umsetzung von Alkanolen und Schwefelwasserstoff in Gegenwart des Katalysators gemäss den Ansprüchen 1 bis 9.

11. Verfahren gemäss Anspruch 10 zur Herstellung von Methylmercaptan durch Umsetzung von Methylalkohol und Schwefelwasserstoff.

## Claims

1. Catalyst comprising a catalytically active tungstate which contains potassium or potassium and caesium as chemically bound alkali metal and tungsten with a molar ratio of alkali metal to tungsten of 1.9:1 to 1:1.

2. Catalyst according to Claim 1, consisting of a coated catalyst in which a support core is coated with the catalytically active tungstate or with a support material impregnated with this tungstate.

3. Catalyst according to Claim 1, in which the support material impregnated with the catalytically active tungstate has been processed to give an unsupported catalyst.

4. Catalyst according to Claim 1, in which the surface of a support body is impregnated with said catalytically active alkali metal tungstate.

5. Catalyst according to Claim 1, **characterized in that** the ratio ranges from 1.6:1 to 1:1.

6. Catalyst according to one or more of Claims 1 to 5, **characterized in that** it comprises the tungstate in an amount of 8 to 45% by weight, preferably 20 to 36% by weight.

7. Catalyst according to one or more of Claims 1 to 6, **characterized in that** the support body or the support material consists of an oxidic inorganic compound.

8. Catalyst according to Claim 7, **characterized in that** the support body or the support material consists of aluminium oxide (Al₂O₃).

9. Catalyst according to Claim 7, **characterized in that** the support material has a specific surface area of 180 to 400 m²/g (BET) and a total pore volume of 0.3 to 1.2 ml/g.

10. Process for preparing alkyl mercaptans by reacting alkanols and hydrogen sulphide in the presence of the catalyst according to Claims 1 to 9.

11. Process according to Claim 10 for preparing methyl mercaptan by reacting methyl alcohol and hydrogen sulphide.

## Revendications

1. Catalyseur contenant un tungstate catalytiquement actif, qui contient, comme métal alcalin lié chimiquement, du potassium ou du potassium et du césium et du tungstène avec un rapport molaire du métal alcalin au tungstène de 1,9:1 à 1:1.

2. Catalyseur selon la revendication 1, constitué par un catalyseur à coquille, dans lequel un noyau support est enrobé par le tungstate catalytiquement actif ou avec un matériau support imprégné de ce tungstate.

3. Catalyseur selon la revendication 1, dans lequel le matériau support imprégné du tungstate catalytiquement actif a été transformé en un catalyseur plein.

4. Catalyseur selon la revendication 1, dans lequel la surface d'un corps support est imprégnée du tungstate de métal alcalin catalytiquement actif mentionné.

5. Catalyseur selon la revendication 1, **caractérisé en ce que** le rapport s'étend de 1,6:1 à 1:1.

6. Catalyseur selon l'une ou plusieurs des revendications 1 à 5, **caractérisé en ce qu'**il contient le tungstate en une quantité de 8 à 45% en poids, de préférence de 20 à 36% en poids.

7. Catalyseur selon l'une ou plusieurs des revendications 1 à 6, **caractérisé en ce que** le corps support ou le matériau support est constitué par un composé inorganique oxyde.

8. Catalyseur selon la revendication 7, **caractérisé en ce que** le corps support ou le matériau support est constitué par de l'oxyde d'aluminium (Al₂O₃).

9. Catalyseur selon la revendication 7, **caractérisé en ce que** le matériau support présente une surface spécifique de 180 à 400 m²/g (BET) et un volume total de pores de 0,3 à 1,2 ml/g.

10. Procédé pour la préparation d'alkylmercaptans par transformation d'alcanols et de sulfure d'hydrogène en présence du catalyseur selon les revendications 1 à 9.

11. Procédé selon la revendication 10 pour la préparation de méthylmercaptan par transformation d'alcool méthylique et de sulfure d'hydrogène.
